(19) Europäisches Patentamt
European Patent Office
Office européen des brevets

(11) **EP 3 705 888 B1**

(12) **EUROPEAN PATENT SPECIFICATION**

(45) Date of publication and mention
of the grant of the patent:
**19.04.2023 Bulletin 2023/16**

(21) Application number: **20160898.1**

(22) Date of filing: **04.03.2020**

(51) International Patent Classification (IPC):
*G01N 33/02* (2006.01)      *G16C 20/30* (2019.01)
*A23B 7/152* (2006.01)      *G01N 33/00* (2006.01)

(52) Cooperative Patent Classification (CPC):
**G01N 33/025; A23B 7/152; G16C 99/00;**
G01N 33/0034; G01N 33/0047; G06N 3/08

(54) **SYSTEM AND METHOD FOR MANAGING RIPENING CONDITIONS OF CLIMACTERIC FRUITS**

SYSTEM UND VERFAHREN ZUR VERWALTUNG VON REIFUNGSBEDINGUNGEN VON
KLIMAKTERISCHEN FRÜCHTEN

SYSTÈME ET PROCÉDÉ DE GESTION DE CONDITIONS DE MATURATION DE FRUITS
CLIMACTÉRIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.03.2019 IN 201921008382**

(43) Date of publication of application:
**09.09.2020 Bulletin 2020/37**

(73) Proprietor: **Tata Consultancy Services Limited
Maharashtra (IN)**

(72) Inventors:
• **Dutta, Jayita**
  **411013 Pune, Maharashtra (IN)**
• **Deshpande, Parijat**
  **411013 Pune, Maharashtra (IN)**
• **Rai, Beena**
  **411013 Pune, Maharashtra (IN)**

(74) Representative: **Goddar, Heinz J.
Boehmert & Boehmert
Anwaltspartnerschaft mbB
Pettenkoferstrasse 22
80336 München (DE)**

(56) References cited:
  **CN-A- 106 973 982      CN-A- 107 703 269
  JP-A- 2018 132 325      US-B2- 7 208 187**

• **BARBOSA NAYARA CANTARINO ET AL:
  "Modeling the respiration rate of Golden papayas
  stored under different atmosphere conditions at
  room temperature", POSTHARVEST BIOLOGY
  AND TECHNOLOGY, vol. 136, 9 November 2017
  (2017-11-09), pages 152-160, XP085264620, ISSN:
  0925-5214, DOI:
  10.1016/J.POSTHARVBIO.2017.11.005**
• **GWANPUA SUNNY GEORGE ET AL: "Modelling
  ethylene regulated changes in 'Hass' avocado
  quality", POSTHARVEST BIOLOGY AND
  TECHNOLOGY, vol. 136, 9 October 2017
  (2017-10-09), pages 12-22, XP085264613, ISSN:
  0925-5214, DOI:
  10.1016/J.POSTHARVBIO.2017.10.002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CROSS-REFERENCE TO RELATED APPLICATIONS AND PRIORITY

The present application claims priority from Indian patent application no. 201921008382, filed on 04 March 2019.

TECHNICAL FIELD

The disclosure herein generally relates to optimal natural ripening conditions of climacteric fruits, and more particularly, to method and system for estimation of optimal natural ripening conditions of climacteric fruits using artificial neural networks (ANN).

BACKGROUND

Increased health awareness has resulted in an increased demand for consumption of fruits. In order to meet the increased demand at the consumer end, local fruit sellers tend to accelerate the ripening process of climacteric fruits by artificial means to reduce the natural ripening period of fruits. Moreover, most of the naturally ripened fruits are not easy to transport and tend to become overripe and inedible when shipped over long distances causing an economic loss and also fails to meet the increased consumer demands.

Conventional solutions for ripening fruits includes home remedies such as presence of emitted ethylene for ripening fruits in an air tight rice container, or spreading unripe fruits in layers over paddy- husk / wheat-straw. On a large scale, artificial ripening is involved to control the ripening rate, enable proper planning in long distance transportation and distribution to customers. For industrial large scale artificial ripening, unripe climacteric fruits are ripened by lighting a smoky fire in an air tight room. Smoke emits various gases like acetylene, ethylene, carbon monoxide which initiate and accelerate ripening process. However, this process of artificial ripening results in non-uniformity with respect to color, flavor and smoky odor and diminishes fruit quality. In another artificial ripening solution, Most export grade climacteric fruits are artificially ripened with industrial grade calcium carbide salt. Calcium carbide when applied on fruits comes in contact of moisture and releases acetylene which also triggers the ripening process. However, industrial-grade calcium carbide usually contains traces of arsenic and phosphorus hydride which causes various health hazards in direct contact. Nayara Cantero Barbosa et al., "Modeling the respiration rate of Golden papayas stored under different atmosphere conditions at room temperature" aims to measure the respiration rate of papayas stored under controlled atmosphere at room temperature, with decreasing $O_2$, and increasing $CO_2$ levels, in order to identify mathematical models capable of predicting respiration rate throughout storage. A model was proposed based on the Michaelis-Menten equation with uncompetitive inhibition and kinetic parameters that change during storage time. A second-order nonlinear regression model was used as reference for the mathematical approach. Nine experiments with three replicates were conducted under different controlled atmospheres to generate respiration data. A closed system method was used to measure the respiration rate at 2 d intervals over 13 d of storage at ambient temperature (23 °C). Peel color measurements indicated total ripening of fruit stored in high $O_2$, atmospheres, whereas ripening was minimal in atmospheres containing low $O_2$ and high $CO_2$ levels. The respiration rate remained at the lowest value, but gradually increased during storage at the lowest $O_2$ level associated with the highest $CO_2$ concentration. The nonlinear regression model obtained the lowest AICc value with VarPow variance, indicating a better fit than the Michaelis-Menten model. However, the latter, whose kinetic parameters change according to a polynomial second-order equation (MMQ), displayed a better fit than the nonlinear regression model evaluated by homoscedastic variance. Additionally, MMQ was more sensitive than nonlinear regression in detecting the real change in respiration rate in a biological system as a function of different gas compositions during storage.

Sunny George Gwanpua et al., "Modelling ethylene regulated changes in 'Hass' avocado quality" states that temperature and exogenous ethylene are two important factors that determine the rate of quality change in 'Hass' avocado. The aim of this study was to quantify this effect and to build a mathematical model that will assist in predicting the effect of temperature, exogenous ethylene, and time on quality outcomes of 'Hass' avocado. A mathematical model was developed to describe autocatalytic ethylene production, and coupled to models describing softening and colour changes via ethylene. The effect of exogenous ethylene was incorporated by addition of a diffusion term, while temperature effect was modelled by expressing rate constants as a function of temperature using the Arrhenius equation. The model was calibrated using data on ethylene efflux rate, firmness and skin hue collected on 'Hass' avocado fruit stored at 5, 10, or 20°C, in atmospheres containing < 0.05 $\mu$L L$^{-1}$ (air), 1 $\mu$L L$^{-1}$ or 10 $\mu$L L$^{-1}$ ethylene in air. The effect of ethylene on softening was greater than on colour change, with clear firmness separation of the different ethylene treated fruit evident even during ripening at 20 °C. The high dependency of softening on ethylene was revealed in the Michaelis-Menten constant for the regulation of the softening enzyme by ethylene, which was about 15 times larger than that for the colour degrading enzymes. The model was successfully validated on a sub-dataset.

SUMMARY

The present invention in the disclosed embodiments presents technological improvements as solutions to one or more of the above-mentioned technical problems recognized by the inventors in conventional systems. The invention discloses a method for managing ripening conditions of climacteric fruit according to claim 1.

The invention also discloses a system for managing ripening conditions of climacteric fruit according to claim 5.

It is to be understood that both the foregoing general description and the following detailed description are exemplary and explanatory only and are not restrictive of the invention, as claimed. The invention is defined by the claims.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a network environment implementing a system for managing natural ripening conditions of climacteric fruits, in accordance with an embodiment of the present subject matter.

FIG. 2 illustrates example system architecture for managing ripening conditions of climacteric fruit, in accordance with an example embodiment.

FIG. 3 illustrates a block diagram of a system for managing the ripening conditions of the climacteric fruits, according to some embodiments of the present disclosure.

FIG. 4 is a flow-diagram of a method for managing natural ripening conditions of climacteric fruits, according to the present invention.

FIG. 5 is a flow diagram of a method for managing natural ripening conditions of climacteric fruits, in accordance with some embodiments of the present disclosure.

FIG. 6A illustrates a variation of O2 consumed and Ethylene emitted by a climacteric fruit (banana) during the natural ripening, in accordance with an example embodiment of the present disclosure.

FIG. 6B illustrates screenshots of life cycle of a natural ripening conditions for a climacteric fruit (banana), in accordance with an example embodiment of the present disclosure.

FIGS. 7A-7E illustrates variations of Ethylene, CO2 released with time for the climacteric fruit (of FIGS. 6A-6B), in accordance with an example embodiment of the present disclosure.

FIGS. 8A and 8B illustrate example GUIs for online, real-time remote monitoring of the environment conditions parameters in enclosure for management of ripening conditions of climacteric fruit, in accordance with an example embodiment.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

Fresh fruits are considered as essential source of nutrients, vitamins, minerals, dietary fibers, sugar/glucose, and the like necessary in human diets for nourishment and their benefits against various chronic diseases such as, different types of cancer, macular and cardiac vascular diseases and other age-related problems. Increased health awareness has resulted in an increased demand for consumption of fruits. In order to meet the increased demand at the consumer end, local fruit sellers tend to accelerate the ripening process of climacteric fruits by artificial means to reduce the natural ripening period.

Most of the naturally ripened fruits are not easy to transport and tend to become overripe and inedible when shipped over long distances causing an economic loss. Once overripe, such fruits fail to meet the increased consumer demands. Moreover, it is quite difficult to predict ripening duration thereof and handle ripe fruits as they are pulpy, fleshy and unstable and hence incur huge losses owing to severe conditions during transportation. Therefore, fruit sellers may prefer to collect mature fruits at an unripe stage directly from the farm and ripen them artificially at destination before selling to customers and thus minimize the losses due to over-ripening and during severe transportation conditions.

All type of fruits cannot be ripened post-harvest and once plucked from plants. Climacteric fruits such as banana, avocado, kiwi, mango, apple, plums, peaches, pears, and the like are plucked from orchards at mature but unripe stage of development so as to allow them to ripen under post-harvest treatment off the plant. However, non-climacteric fruits such as oranges, grapes, watermelons, litchis, strawberries, raspberries, cherries, and the like are incapable of handling post-harvest treatment and continuing their natural ripening process when removed from plants. Climacteric fruits naturally emit ethylene over time, responsible for the natural ripening process but climacteric fruits and non-climacteric fruits respond differently to emitted ethylene or external ethylene exposure. Non-climacteric fruits produce very small quantities of ethylene and do not respond to external ethylene treatment to allow ripening except for the color change off the plant. Hence, they are harvested only when they are completely ripe and ready to be consumed. However, climacteric fruits produce comparably larger quantities of ethylene and respond to external ethylene exposure in terms of ripening externally, post-harvest off the plant. Moreover, small amount of ethylene can accelerate the ripening process of climacteric fruits under controlled conditions of temperature and humidity.

Despite the aforementioned challenges, a complete uniform climacteric fruit ripening solution is unavailable. Some existing home remedies such as natural ripening solutions in presence of emitted ethylene like small scale mango ripening in an air tight rice container or spreading unripe fruits in layers over paddy- husk / wheat-straw are widely practiced. However, these ripening solutions do not provide quick accurate or uniform ripening and may extend up to several days affecting the quality and taste of the climacteric fruits. For large scale production and marketing of climacteric fruits commercially, artificial ripening is involved to control the ripening rate, enable proper planning in long distance transportation and distribution to customers. For industrial, large scale artificial ripening, unripe climacteric fruits are ripened by lighting a smoky fire in an air tight room. Smoke emits various gases like acetylene, ethylene, carbon monoxide which initiate and accelerate ripening process. However, this process of artificial ripening results in non-uniformity with respect to the color, flavor and smoky odor and diminishes fruit quality. Similarly, Ethephon and/or Ethrel are also used as ripening agents which releases ethylene depending on the fruit pH and relative humidity. Most export grade climacteric fruits are artificially ripened with industrial grade calcium carbide salt but it is hundred times less effective as compared to Ethylene. Calcium carbide when applied on fruits comes in contact of moisture and releases acetylene which also triggers the ripening process. However, industrial-grade calcium carbide usually contains traces of arsenic and phosphorus hydride which causes various health hazards in direct contact. Acetylene is believed to affect the central nervous system by reducing oxygen supply to brain and hence use of calcium carbide for artificial ripening are banned in most countries.

An effective and safe ripening solution is use of ethylene gas as it does not affect human health adversely even when consumed in large quantities over a long period of time. Export grade climacteric fruits are exposed to ethylene gas in air tight chambers to ensure de-greening and ripening of climacteric fruits and to retain the sweetness, aroma and quality of the fruit. But pure ethylene gas being flammable in nature, when kept in pressurized chambers, tends to increase the risk of explosion. Based on the aforementioned, it can be concluded that the best ripening solution is natural ripening of climacteric fruits post-harvest in presence of naturally emitted ethylene which may act as a natural hormone for ripening under controlled temperature and relative humidity conditions.

Various embodiments described herein provides method and system to naturally ripen climacteric fruits in presence of emitted ethylene from the unripe fruits kept in a cost effective, custom enclosure where environmental conditions such as temperature, relative humidity, CO2, O2 can be controlled and altered. In an embodiment, the disclosed method includes real-time remote monitoring of natural fruit ripening process that facilitates in estimating an optimal ripening solution for climacteric fruits. Additionally, the system implements cyclic variation of temperature and light exposure to the climacteric fruits to mimic the diurnal pattern of the fruit. In an embodiment, the disclosed system includes an IoT enabled, integrated system where a collection of select sensors such as Temperature, Humidity, Ethylene, O2, CO2 and high resolution optical cameras illuminated at various wavelengths of visible light are combined to quantitatively sense and continuously monitor the natural ripening process of climacteric fruits in the presence of ethylene emitted over time under controlled temperature and relative humidity conditions.

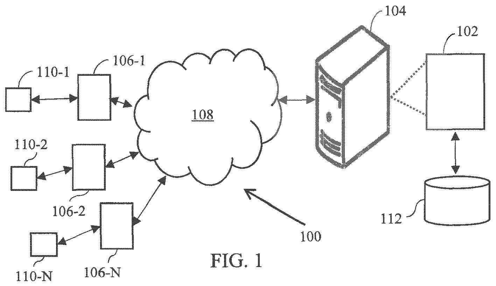

In an embodiment, the disclosed system is capable of quantitatively sense and measure naturally emitted ethylene and CO2 and consumed O2 under user defined conditions of temperature and humidity along with high resolution images of the climacteric fruit that may be recorded during various stages ripening of the climacteric fruit. In an embodiment, the system may include an artificial neural network (ANN) that is trained on data pertaining to the naturally emitted ethylene and CO2 and consumed 02, and high resolution images corresponding to various stages of ripening of the climacteric fruit. The trained ANN model facilitates in estimating and/or predicting optimal ripening conditions for naturally ripening the climacteric fruits. The system is capable of tuning the environmental conditions in the chamber so as to customize/alter number of days required for natural ripening of the climacteric fruits. A detailed description of the above described embodiments for estimating optimal natural ripening conditions of climacteric fruits is presented with respect to illustrations represented with reference to FIGS. 1 through 7E.

Exemplary embodiments are described with reference to the accompanying drawings. In the figures, the leftmost digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from scope of the disclosed embodiments. It is intended that the following detailed description be considered as exemplary only, with the true scope being indicated by the claims.

Referring now to the drawings, and more particularly to FIG. 1 through 7E, where similar reference characters denote corresponding features consistently throughout the figures, there are shown preferred embodiments and these embodiments are described in the context of the following exemplary system and/or method.

FIG. 1 illustrates a network environment 100 implementing a system 102 for managing natural ripening conditions of climacteric fruits, in accordance with an embodiment of the present subject matter.

In an embodiment, the system 102 may receive sensory data and visual data during monitoring and estimation of the climacteric fruit from a networked framework, for example a networked framework 104. The framework may be embodied in an enclosure, as will be described later. Herein, it will be understood that said enclosure is configured to incorporate multiple mutually exclusive sensors, including but not limited to, optical sensor, Temperature, Humidity, Ethylene, O2 and CO2, and so on integrated inside a customized enclosure configured to perform periodic, synchronized data logging corresponding to the climacteric fruit. In an embodiment, said mutually exclusive sensors may be integrated in a smart plate.

The aforementioned enclosure enables multivariate sensing and monitoring of climacteric fruits such as banana, avocado, kiwi, mango, apple, plums, peaches, pears, and other such climacteric fruits. In an embodiment, the sensors are modular and may be replaced based upon the food item. In an embodiment, the disclosed enclosure may be a modular enclosure where any existing sensor can be removed or any new sensor can be plugged in as per requirements. Also, multiple sensors and multiple image capturing devices (such as cameras) may be installed inside the custom enclosure with parameter variation control like temperature, humidity, and so on to capture periodic changes in the climacteric fruit from all directions depending upon the requirements. A suitable enclosure for estimating ripening conditions of the climacteric fruits is further described in the Indian Patent application no. 201821040783 titled, "Integrated Framework for Multimodal Sensing and Monitoring of Perishable Items".

In an embodiment, the framework may be an IoT enabled integrated system/framework capable of real time collection of high resolution images along with quantitative sensing and remote monitoring of ethylene and CO2 emitted and O2 consumed over time when the climacteric fruit is kept in the enclosure under controlled temperature and relative humidity. The climacteric fruit may be kept inside the enclosure under controlled temperature and relative humidity to allow the natural process of fruit ripening in the presence of emitted Ethylene over time. Multimodal sensing suite, integrated with a collection of select sensors such as Temperature, Humidity, Ethylene, O2 and CO2 may be mounted inside the custom enclosure to enable continuous quantitative sensing and monitoring of periodic changes in the climacteric fruit over time with respect to changes in emitted ethylene, CO2 gas levels and consumed O2 inside the enclosure. Multiple high resolution optical cameras are installed at different directions inside the enclosure and are illuminated at various wavelengths of visible light to capture pictorial variations, continuously and automatically at periodic intervals, in the climacteric fruits from multiple directions and witness the periodic changes from unripe to ripening stage in terms of colour, texture, and so on. The custom enclosure is further capable of regulating temperature and relative humidity, thus allowing recreating the environmental conditions similar to that experienced by the climacteric fruits post-harvest in the real field or during storage or in-transit.

Due to the aforementioned, the disclosed system 102 is thus capable of supporting controlled variation in ripening conditions as per the selected climacteric fruit and obtain continuous and automatic quantitative synchronized data streams from various sensors and high resolution images from multiple cameras, periodically, to monitor the natural ripening process and enabling an optimal ripening solution. Further, the system 102 also supports selective ventilation within the enclosure, for example, with the help of small exhaust fan to allow adequate airflow to guarantee circulation of ethylene necessary for the natural ripening of unripe climacteric fruits and also prevent accumulation of excessive ethylene and CO2 levels within the enclosure which can in turn result in over-ripening of fruits.

Although the present disclosure is explained considering that the system 102 is implemented on a server, it may be understood that the system 102 may also be implemented in a variety of computing systems, such as a laptop computer, a desktop computer, a notebook, a workstation, a cloud-based computing environment and the like. It will be understood that the system 102 may be accessed by multiple contributors through one or more devices 106-1, 106-2... 106-N, collectively referred to as devices 106 hereinafter, or applications residing on the devices 106. Examples of the devices 106 may include, but are not limited to, a portable computer, a personal digital assistant, a handheld device, a smartphone, a tablet computer, a workstation and the like. The devices 106 are communicatively coupled to the system 102 through a network 108.

In an embodiment, the network 108 may be a wireless or a wired network, or a combination thereof. In an example, the network 108 can be implemented as a computer network, as one of the different types of networks, such as virtual private network (VPN), intranet, local area network (LAN), wide area network (WAN), the internet, and such. The network 106 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 208 may include a variety of network devices, including routers, bridges, servers, computing devices, storage devices. The network devices within the network 108 may interact with the system 102 through communication links.

As discussed above, the system 102 may be implemented in a computing device 104, such as a hand-held device, a laptop or other portable computer, a tablet computer, a mobile phone, a PDA, a smartphone, and a desktop computer. The system 102 may also be implemented in a workstation, a mainframe computer, a server, and a network server. In an embodiment, the system 102 may be coupled to a data repository, for example, a repository 112. The repository 112 may store data processed, received, and generated by the system 102. In an alternate embodiment, the system 102 may include the data repository 112.

In an embodiment, the network environment 100 may be an internet of things (IoT) based environment having various hardware and software elements collectively configured to perform real-time data analytics in the smart computing environment, according to an exemplary embodiment of the disclosure. The IoT based platform backend may include a cloud server, for example the server 104 connected to a database, for example, the database 112. The system 100 further includes various IoT based devices, for example the devices 106 implemented on different smart devices such as smart phone, a telematics device, and so on enabling real-time analytics of sensor data. The system further includes various heterogeneous sensor devices, for example sensor devices 110-1, 110-2, 110-N (hereinafter collectively referred to as sensor devices 110) and so on, placed in the vicinity of smart computing environment connected with various IoT based devices 106. Alternatively, said sensor devices 110 may be embodied in the IoT based devices 106. Thus, the sensor devices 110 along with the IoT based devices 106 may collectively form an intelligent smart environment according to this exemplary embodiment.

In an embodiment, the system 102 includes an artificial neural network (ANN) that may be pre-trained using multi-variate, multi-parameter, multi-modal sensory data as well as visual data associated with climacteric fruit kept inside the enclosure. In real-time, the system 102 receives the sensory data and the visual data from the integrated framework monitoring the food item, and the ANN estimates/predicts a ripening condition/stage of the climacteric fruit by utilizing the ANN model. The ANN further estimates a number of days remaining for complete ripening of the climacteric fruit.

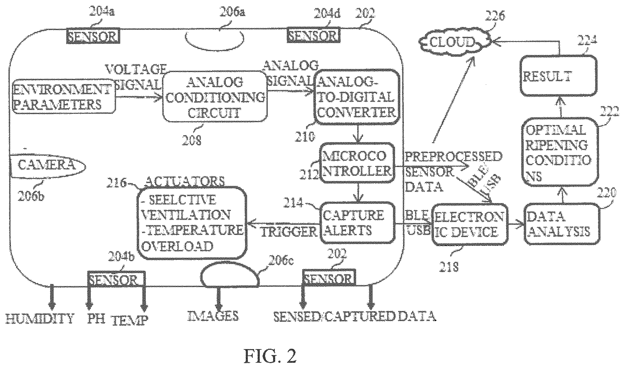

Further, as illustrated in FIG. 1, the network environment 100 supports various connectivity options such as BLUETOOTH®, USB, ZigBee and other cellular services. In an exemplary embodiment, the system 102 interfaces with sensors 110 such as GPS, accelerometers, magnetic compass, audio sensors, camera sensors, and so on. Based on the data collected from various sensors, the system 102 with the help of various hardware and software platforms, collectively performs the task of scalable data analytics on the captured sensor data in any smart computing environment. The network environment enables connection of devices 106 such as Smartphone with the server 104, and accordingly with the database 112 using any communication link including Internet, WAN, MAN, and so on. In an exemplary embodiment, the system 102 is implemented to operate as a stand-alone device. In another embodiment, the system 102 may be implemented to work as a loosely coupled device to the smart computing environment. The components and functionalities of the system 102 are described further in detail with reference to FIGS. 2. An example of a process flow for utilizing said framework for managing ripening condition of climacteric fruit is described further with reference to FIG. 2.

FIG. 2 illustrates example system architecture 200 for managing ripening conditions of climacteric fruit, in accordance with an example embodiment. Herein, the system architecture is pertaining to the system, for example the system 102 (FIG. 1). As disclosed previously, the system is implemented for real time estimation of ripening conditions of climacteric fruit during storage and at transit. Furthermore, is able to predict the ripening time from a given stage of a climacteric fruit by using an ANN model. Accordingly, the system alters the environmental conditions of the climacteric fruits so that said fruits can be made to ripen in the desired time. Based on the ANN model, the system predicts the number of days needed for a particular grade of climacteric fruits such as banana to ripen. In an instance, the system can predict that with the current ripening kinetics, the banana ripens in upcoming 10 days. But, at this stage if said banana is to be ripened in 8 days, then the environment condition parameters such as Temperature, Relative Humidity, and the like can be altered inside the enclosure such that the ripening kinetics of the banana changes and it gets ripened naturally in 8 days instead of 10 days.

In an embodiment, the system may be embodied in an enclosure, for instance an enclosure 202. Alternatively, the system may be communicatively coupled to the enclosure 202. The enclosure 202 may include multiple smart plates installed therein where environment condition parameters at source or during storage or at transit can be recreated. For example, as illustrated the enclosure includes sensor suites (each suite having multiple sensors) such as sensor suites 204a, 204b, 204c, and 204d (hereinafter referred to as sensor suites 204), and multiple media capturing devices/sensors such as devices 206a, 206b, 206c, 206d (hereinafter referred to as devices 206). The sensors of the sensor suites 204 along with the devices 206 captures sensory data and visual data, respectively pertaining to the climacteric fruit. Said extensive synchronized data collection is followed by data filtration and data preprocessing periodically.

In an embodiment, the system 200 may include a signal processing components and circuitry for preprocessing (hereinafter referred to as preprocessing circuitry) the visual data and the sensor data associated with the monitoring of the climacteric fruit received from the enclosure. In an embodiment, the preprocessing circuitry may be embodied in the system. Alternatively, the preprocessing circuitry may not be embodied in the system, instead the circuitry may be embodied in the framework for preprocessing the visual/image data, and the system may receive said preprocessed data from the framework.

As illustrated in FIG. 2, sensed raw signals from the sensors may be fed to a signal isolation circuit in order to remove induced noise from the sensor signal(s), prevent ground looping in the communication network and provide proper isolation between the sensor signals. An analog signal conditioning and data preprocessing circuit 208 performs signal conditioning, which involves filtering of unwanted frequencies. In an embodiment, analog signal conditioning and data preprocessing circuit 208 includes an antialiasing filter to prevent overlapping of signals and reduce high frequency noise. A preamplifier increases the signal strength and improves the gain of the signal followed by Voltage to Frequency converter followed by Binary Coded Decimal (BCD) counter for counting of frequency and thus converts voltage signal to analog signal. The analog output is then converted to digital signal via analog to digital converter circuit (ADC) 210. The digital data is then processed via a microcontroller unit 212 for further applications which require quantifiable data for estimating ripening condition of the climacteric fruit. For example, the preprocessed data can be utilized for pre-processed data logging and capturing of alerts beyond permissible thresholds of the sensed parameter. The captured alerts 214 may trigger the actuator 216 to bring back the system to desired condition in case of temperature overload or excessive built-up of gases in the enclosure. The pre-processed data along with captured alerts is interfaced to electronic devices 218 such as a personal computer (PC) and/or laptop and/or tablets and/or smart phones via for instance, Bluetooth Low Energy (BLE) module or via USB which serves as offline data storage for further data analysis (220) of the natural ripening process and enable user to estimate optimal ripening conditions 222. Herein, the framework may be IoT enabled, thereby enabling storage of result 224 of the data analysis on a cloud 226 via for instance, Wi-Fi module and permits remote data access via for instance, internet.

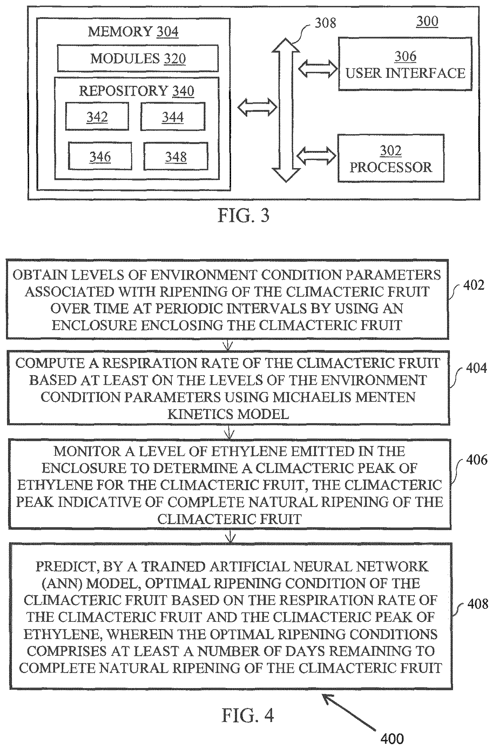

The interfacing may be utilized for data analysis. Said data analysis facilitate in finding data correlations (or data analysis), via artificial neural network (ANN), thereby leading to a customized digital twin for monitoring and estimating ripening conditions of the climacteric fruits. Herein, the digital twin refers to an ANN based model that can predict the ripening conditions of the climacteric fruits. In order to predict the ripening conditions of the climacteric fruits, the system monitors and records the ethylene emitted from unripe fruits, when kept inside the enclosure under selective ventilation and controlled environment. The ethylene emitted from unripe fruits triggers and accelerates the process of natural ripening for unripe climacteric fruits. The system records combined effect of naturally emitted ethylene gas levels, $CO_2$ and consumed $O_2$ under predefined conditions of temperature and humidity and facilitate in estimating an optimal ripening solution by varying ripening conditions. In an embodiment, the disclosed system for monitoring and estimation of ripening conditions of the climacteric fruits is described further with reference to FIG. 3.

FIG. 3 illustrates a block diagram of a system 300 for managing the ripening conditions of the climacteric fruits, according to some embodiments of the present disclosure. The system 300 may be an example of the system 102 (FIG. 1). The system 300 provides continuous and quantitative sensing of variations of the climacteric fruit kept inside a controlled enclosure with respect to gases emitted from the climacteric fruit (such as ethylene, $O_2$, $CO_2$) and image changes of said fruit.

As previously described, the system 300 may be embodied or housed in a custom enclosure, capable of controlling the temperature and humidity inside the enclosure and also allow variation of these parameters as per requirement to achieve natural ripening conditions of climacteric fruits during a stipulated time frame. Natural ripening process in the presence of emitted ethylene from the climacteric fruit itself, needs adequate air flow inside the enclosure to ensure circulation of ethylene but higher concentration of ethylene in the enclosure results in over-ripening and fast degradation of the fruits. To avoid such over-ripening, the enclosure provisions selective ventilation of emitted ethylene, $CO_2$ and $O_2$ and intake of fresh air. Thus, the system 300 allows generation of alerts when concentrations of these gases exceed or is less than the value of a predefined threshold and facilitates venting of excess gases from the enclosure followed by inclusion of fresh air.

The system 300 includes one or more hardware processors such as a processor 302, at least one memory such as a memory 304, and a communication interface 306. The processor 302, memory 304, and the communication interface 306 may be coupled by a system bus such as a system bus 308 or a similar mechanism. The communication interface 306 may include an I/O interface that may have a variety of software and hardware interfaces, for example, a web interface, a graphical user interface, and the like The interfaces 306 may include a variety of software and hardware interfaces, for example, interfaces for peripheral device(s), such as a keyboard, a mouse, an external memory, and a printer. Further, the interfaces 306 may enable the system 300 to communicate with other devices, such as web servers and external databases. The interfaces 306 can facilitate multiple communications within a wide variety of networks and protocol types, including wired networks, for example, local area network (LAN), cable, and so on, and wireless networks, such as Wireless LAN (WLAN), cellular, or satellite. For the purpose, the interfaces 306 may include one or more ports for connecting a number of computing systems with one another or to another server computer. The interface 306 may include one or more ports for connecting a number of devices to one another or to another server.

The hardware processor 302 may be implemented as one or more microprocessors, microcomputers, microcontrollers, digital signal processors, central processing units, state machines, logic circuitries, and/or any devices that manipulate signals based on operational instructions. Among other capabilities, the hardware processor 302 is configured to fetch and execute computer-readable instructions stored in the memory 304.

The memory 304 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 304 includes a plurality of modules 320 and a repository 340 for storing data processed, received, and generated by one or more of the modules 320. The modules 320 may include routines, programs, objects, components, data structures, and so on, which perform particular tasks or implement particular abstract data types. The repository 340, amongst other things, includes a system database 342 and other data 344. The other data 344 may include data generated as a result of the execution of one or more modules in the modules 320. The repository 340 may further include a sensory data 346 and image data 348 obtained during the monitoring and estimation of climacteric fruits, as will be explained further in detail below.

The system 300 is configured to perform monitoring and estimation of ripening conditions of the climacteric fruits periodically. The system 300 may receive images of climacteric fruit and always receives sensory data consisting of values or levels of temperature, relative humidity, CO2, O2, ethylene from a plurality of sensors housed within the enclosure for synchronized, continuous monitoring of the quantitative variations in the select climacteric fruit over time at periodic intervals.

In an embodiment, the system 300 may receive sensory data 346 and image data 348 from the plurality of sensors (as explained with reference to FIG. 2) house in the enclosure. In an embodiment, the sensory data provide levels of environment condition parameters associated with ripening of the climacteric fruit over time at periodic intervals by using an enclosure enclosing the climacteric fruit. In the present invention, the environment condition parameters include CO2 emitted, O2 consumed, Ethylene emitted, temperature and relative humidity measured within the enclosure.

The system 300 preprocesses the sensory data 346 and the image data 348. In an embodiment, the one or more hardware processors may facilitate preprocessing of the sensory data 346 and the image 348, as explained with reference to FIG. 2.

The system 300 computes a respiration rate of the climacteric fruit based at least on the levels of the environment condition parameters using Michaelis Menten kinetics model. The Menten Michaelis kinetics model captures change in biochemistry of the climacteric fruit. In other words, the Menten Michaelis kinetics model captures the enzyme kinetics of the climacteric fruit. According to Enzyme kinetics, decrease in % Relative Humidity and increase in temperature, increases the Respiration rate of the climacteric fruit and results in faster ripening.

The Menten Michaelis kinetics model is based on the fact that the selected samples of fruits and vegetables are alive and therefore have a specific respiration rate. Hence, said fruits/vegetables result in creating a particular environment condition in the enclosure. Said environment condition can be represented by environment condition parameters such as CO2, O2, relative humidity and temperature. The levels of the environment condition parameters facilitates in computing respiration rate of the climacteric fruit. In an embodiment, excessive buildup of gases such as CO2, O2 and humidity is limited by the selective ventilation provided in the enclosure.

In an embodiment, the respiration rate of the climacteric fruit is expressed in terms of O2 consumption rate and CO2 production rates in the enclosure. The respiration rate or the O2 consumption rate and CO2 production rates may vary depending on the environment and the presence of inhibitors such as high levels of CO2 or Humidity within the enclosure.

CO2 inhibition can be of five types namely no inhibition (1), competitive(2), uncompetitive(3), non-competitive(4) and combination of competitive and uncompetitive(5) and can represented by Michaelis-Menten (MM) equation as mentioned below -

$$R = (\alpha \times yO2)/(\varphi + yO2) \ \ ----(1)$$

$$R = (\alpha \times yO2)/ (\varphi \times (1 + yCO2/yc) + yO2) \ ---(2)$$

$$R = (\alpha \times yO2)/ (\varphi + (1 + yCO2/yu) \times yO2) \ ----(3)$$

$$R = (\alpha \times yO2)/ (\varphi + yO2) \times (1 + yCO2/yn) \ ----(4)$$

$$R = (\alpha \times yO2)/ ((\varphi \times (1 + yCO2/yc) )+ ( yO2 \times(1 + yCO2/yu) ) ) \ ----(5)$$

where , R is the respiration rate,

$y_{O_2}$ and $y_{CO_2}$ are oxygen and carbon dioxide concentrations (%),

$\alpha$ is the maximum $O_2$ consumption rate,

$\varphi$ is the Michaelis constant for $O_2$ consumption (%),

$y_c$ the Michaelis constant for the competitive inhibition of $O_2$ consumption by $CO_2$ (%), and

$y_u$ the Michaelis constant for the uncompetitive inhibition of $O_2$ consumption by $CO_2$ (%).

In an embodiment, the system may estimate the Michaelis Menten coefficients ($\alpha$, $\varphi$, $y_u$, $y_c$) using samples of the climacteric fruit by converting it into linear regression model. Mathematical technique used in estimating the regression parameters of the model may include least square method where the sum of squared errors between observed values and predicted values is minimized. The estimated coefficients predict the respiration rates and therefore the enzymatic kinetics of the sample.

In the present invention, the system 300 computes the respiration rate of the climacteric fruit based on the levels of the environment condition parameters and the estimated Michaelis Menten coefficients.

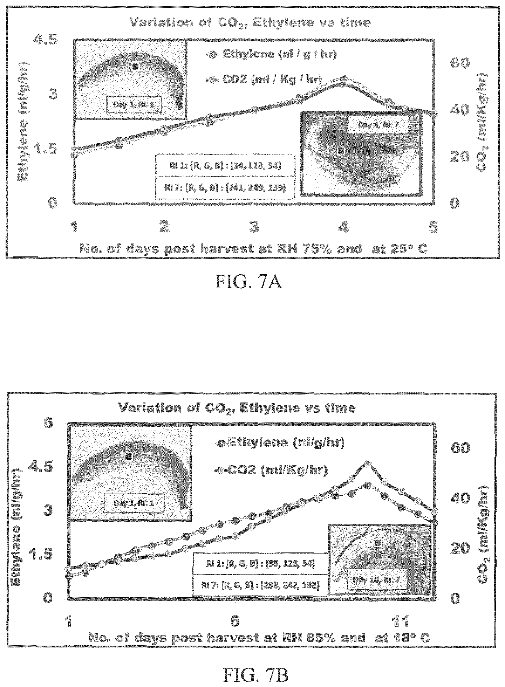

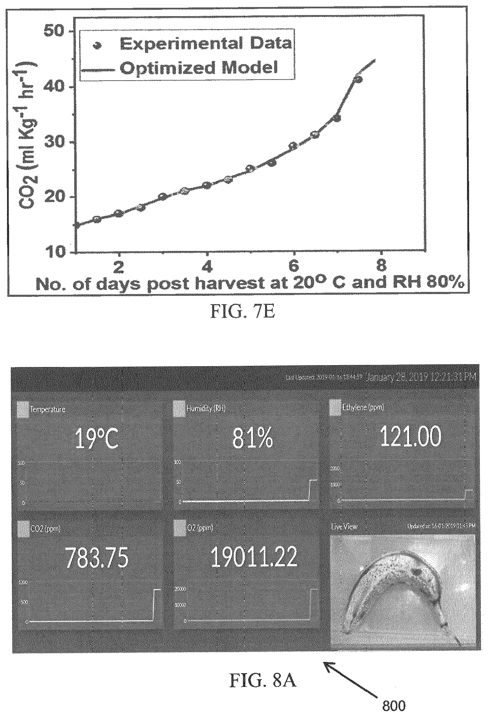

The fully ripened condition of the climacteric fruit can be identified by the peak of ethylene levels in the enclosure. Ethylene is known as the fruit ripening hormone and different level of ethylene is produced by every climacteric fruit during different stages of its life cycle. Ethylene concentration gradually increases in any climacteric mature, unripe fruit, such as the case of a banana and triggers the fruit's metabolism, accelerating the ripening process naturally. The increase in ethylene concentration reaches a peak beyond which it starts to decrease. The ethylene concentration increases under controlled environment, increasing the ripening rate of the climacteric fruit in pre-climacteric phase gradually changing the color of the fruit (for example, de-greening the color of banana to yellowish), followed by attaining a climacteric peak when the concentration of ethylene is maximum and the climacteric is completely ripened with aroma. Also, the color of the fruit changes, for example that of banana changes to yellow with brown spots. Beyond the climacteric peak, ethylene concentration gradually decreases in the post climacteric phase when the fruit starts to over ripen and gets gradually degraded, turning to black in color. An example of life cycle of natural ripening condition of a climacteric fruit in terms of Ethylene and $CO_2$ released is described in detail with reference to FIGS. 7A and 7E.

In the present invention, the system monitors a level of Ethylene emitted in the enclosure to determine a climacteric peak of Ethylene for the climacteric fruit. As discussed above, the climacteric peak indicative of complete natural ripening of the climacteric fruit. Based on the climacteric peak of ethylene and the respiration rate of the climacteric fruit, the system 300 predicts optimal ripening condition of the climacteric fruit. In the present invention, the optimal ripening condition corresponding to a climacteric fruit includes at least a number of days remaining to complete natural ripening of the climacteric fruit.

The system 300 includes a pre-trained artificial neural network (ANN) model to predict the optimal ripening condition of the climacteric fruit. The ANN model may be pre-trained for prediction based on a plurality of features. Examples of said features includes, but not limited to, $CO_2$ emitted concentration over time, Ethylene emitted concentration over time, difference of $O_2$ in the enclosure after a predefined interval, Temperature, Relative humidity, average respiration rate, average temperature, average humidity, average ethylene rate, ethylene concentration at the climacteric peak, and $CO_2$ concentration at the climacteric peak.

In an embodiment, the training of the ANN model may include continuous monitoring of the sample of climacteric fruit to determine Ethylene concentration and $CO_2$ concentration emitted therefrom over time. In an example embodiment, the emitted Ethylene concentration and $CO_2$ concentration over time is calculated using the formula given below -

$$r_{C_2H_4}(t) = (V/W)(y_{C_2H_4\,t+1} - y_{C_2H_4\,t-1})/\Delta t$$

$$r_{CO_2}(t) = (V/W)(y_{CO_2\,t+1} - y_{CO_2\,t-1})/\Delta t$$

where, $r_{C_2H_4}(t)$ and $r_{CO_2}(t)$ are emitted ethylene and $CO_2$ concentration over time respectively.

V is the volume of the enclosure

$\Delta t$ is the periodic interval at which synchronized data streams (including sensor data and image data) are collected

$y_{C_2H_4\,t+1}$ and $y_{C_2H_4\,t-1}$ are concentration of Ethylene at time t+1 and at time t-1.

$y_{CO_2\,t+1}$ and $y_{CO_2\,t-1}$ are concentrations of $CO_2$ at time t+1 and t-1 respectively.

The ANN model captures Ethylene concentration and $CO_2$ concentration over time and during said time, the climacteric fruit undergoes various stages of natural ripening process. The ANN model further obtains annotations to classify different stages of fruit ripening. The ANN model may obtain said annotations from, for instance experts from the fruit industry, published annotations based on pictures, and so on, corresponding to various stages of the natural ripening process. Depending on the annotations (images or pictorial representation of different ripening stages of climacteric fruit), said climacteric fruits are categorized in terms of Ripening Index (RI). The RI may be defined as an index that is capable of quantitatively predicting the natural ripening of the climacteric fruit based on the Ethylene emitted and respiration rate of the climacteric fruit.

Further, the system performs RGB image analysis on different pictorially reported ripening stages of the climacteric fruits by considering said images as standard annotation to determine the average content of Red, Green and Blue color content from the images of the climacteric fruit and find the resultant of the three parameters. In an embodiment, said RGB image analysis can be performed using MATLAB.

In the present invention, the system utilizes the ANN to predict the optimal ripening condition of the climacteric fruit by identifying stage of ripening of the climacteric fruit associated with a co-occurrence of the climacteric peak of ethylene emitted and a zero rate of change of the respiration rate. Since the model predicts the optimal ripening conditions (in terms of ethylene, $CO_2$, number of days) of the climacteric fruit through its different ripening stages, the system 300 alters the environment conditions in the enclosure to obtain the predicted optimal ripening conditions, and thus a desired ripening duration. In an embodiment, altering the environment conditions includes performing at least one of varying the temperature and the relative humidity of the enclosure, and ventilating excess $CO_2$ and Ethylene when the levels of $CO_2$ and ethylene reaches peak. For example, once the ANN model predicts the number of days needed for a particular grade of the climacteric fruit to ripen and attain the climacteric peak, the ripening kinetics (obtained from the environment condition parameters) of a new sample of the same climacteric fruit can be obtained at periodic intervals (using the enclosure). Using the ripening kinetics as input to the ANN model, the system can predict that the climacteric fruit may ripen in upcoming 10 days. However, at this stage if the climacteric fruit is to be ripened in 8 days, the system can vary the temperature, relative humidity inside the enclosure such that the ripening kinetics of the climacteric fruit changes and it gets ripened in 8 days instead of 10 days. A flow-diagram describing a method for managing ripening of the climacteric fruits is described further with reference to FIG. 4.

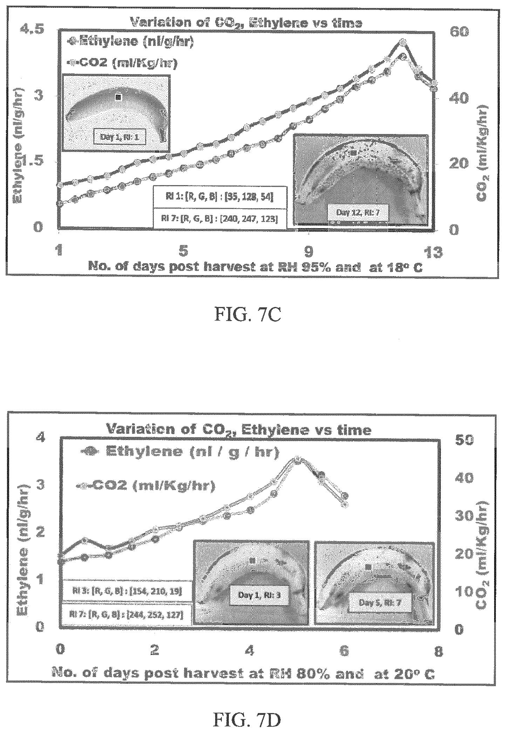

Referring now to FIG. 4, a flow-diagram of a method 400 for managing natural ripening conditions of climacteric fruits, according to the present invention. The method 400 may be described in the general context of computer executable instructions. Generally, computer executable instructions can include routines, programs, hands, components, data structures, procedures, modules, functions, etc., that perform particular functions or implement particular abstract data types. The method 400 may also be practiced in a distributed computing environment where functions are performed by remote processing devices that are linked through a communication network. The order in which the method 400 is described is not intended to be construed as a limitation, and any number of the described method blocks can be combined in any order to implement the method 400, or an alternative method. Furthermore, the method 400 can be implemented in any suitable hardware, software, firmware, or combination thereof. In an embodiment, the method 400 depicted in the flow chart may be executed by a system, for example, the system 102 of FIG. 1. In an example embodiment, the system 102 may be embodied in an exemplary computer system, for example computer system 701 (FIG. 7). The method 400 of FIG. 4 will be explained in more detail below with reference to FIGS. 1-3.

Referring to FIG. 4, at 402 the method 400 includes obtaining levels of environment condition parameters associated with ripening of the climacteric fruit over time at periodic intervals by using an enclosure enclosing the climacteric fruit, the environment condition parameters comprising $CO_2$ emitted, $O_2$ consumed, Ethylene emitted, temperature and relative humidity measured within the enclosure. At 404, the method 400 includes computing a respiration rate of the climacteric fruit based at least on the levels of the environment condition parameters using Michaelis Menten kinetics model. At 406, the method 400 includes monitoring a level of Ethylene emitted in the enclosure to determine a climacteric peak of Ethylene for the climacteric fruit, the climacteric peak indicative of complete natural ripening of the climacteric fruit. At 408, the method 400 includes predicting, by a pre-trained ANN model, optimal ripening condition of the climacteric fruit based on the respiration rate of the climacteric fruit and the climacteric peak of ethylene, wherein the optimal ripening conditions comprises at least a number of days remaining to complete natural ripening of the climacteric fruit.

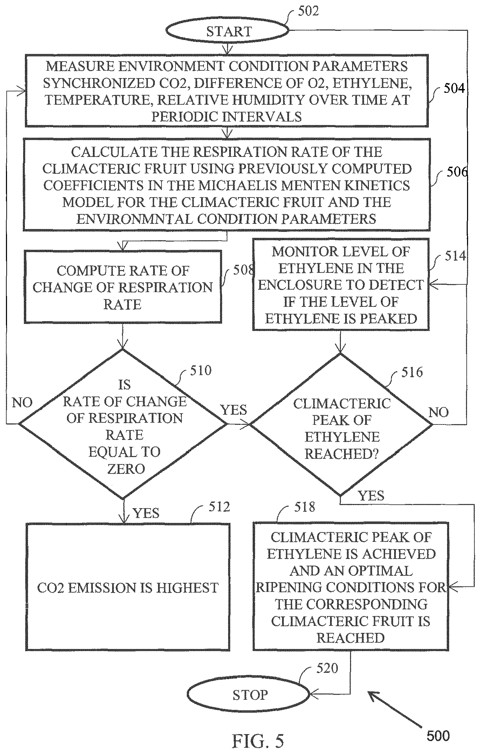

Referring now to FIG. 5, a flow-diagram of a method 500 for managing natural ripening conditions of climacteric fruits, according to some embodiments of present disclosure. The method 500 may be described in the general context of computer executable instructions. Generally, computer executable instructions can include routines, programs, hands, components, data structures, procedures, modules, functions, etc., that perform particular functions or implement particular abstract data types. The method 500 may also be practiced in a distributed computing environment where functions are performed by remote processing devices that are linked through a communication network. The order in which the method 500 is described is not intended to be construed as a limitation, and any number of the described method blocks can be combined in any order to implement the method 500, or an alternative method. Furthermore, the method 500 can be implemented in any suitable hardware, software, firmware, or combination thereof. In an embodiment, the method 500 depicted in the flow chart may be executed by a system, for example, the system 102 of FIG. 1. In an example embodiment, the system 102 may be embodied in an exemplary computer system, for example computer system 701 (FIG. 7). The method 500 of FIG. 5 will be explained in more detail below with reference to FIGS. 1-3.

Referring now to FIG. 5, the method 500 is initiated at 502. At 504, the method 500 includes measuring environment parameters such as synchronized CO2, difference of O2, ethylene, temperature, relative humidity over time at periodic intervals. In an embodiment, said environment parameters may be measured by utilizing the enclosure, for example the enclosure 202 (FIG. 2). As previously discussed, the enclosure includes a plurality of sensors for measuring the environment parameters. At 506, the method 500 includes calculating the respiration rate of the climacteric fruit using the environment parameters and previously computed coefficients in the Michaelis Menten kinetics model for the climacteric fruit. The Michaelis Menten kinetics model and computation of the respiration rate (for exmaple, $R_{CO2}$) is previously described in detail with reference to FIG. 3.

At 508, a rate of change of respiration rate ($dR_{CO2}/dt$) is computed. The rate of change of respiration rate is indicative of CO2 emission or O2 consumption in the enclosure. At 510, it is determined whether the rate of change of respiration rate is equal to zero. A zero value of the rate of change of the respiration rate of the climacteric fruit enclosed in the enclosure is indicative of a maximum emission of CO2 (thereby meaning maximum respiration rate). If, at 510, it is determined that the rate of change of respiration rate is not equal to zero the level of environment condition parameters is computed again at 504. If however, at 510, it is determined that the rate of change of respiration rate is equal to zero, at 512, it is concluded that the CO2 emission is highest.

At 514, the level of ethylene in the enclosure is monitored to determine if the level of ethylene is peaked. In an embodiment, the peak of ethylene is determined by determining a rate of change of ethylene with respect to time. A zero rate of change of ethylene with respect to time indicates that the ethylene has peaked. At 516, it is determined whether climacteric peak of the ethylene is reached. In an embodiment, the level of ethylene in the enclosure is monitored for peak till the climacteric peak of ethylene is reached. If however it is determined at 516 that the climacteric peak of ethylene is reached, then at it is concluded at 518 that optimal ripening conditions for the corresponding climacteric fruit is reached. It will be understood here that the optimal ripening conditions for a climacteric fruit is determined to be reached (1) when a rate of change of respiration rate is equal to zero (which implies that maximum amount of CO2 is being emitted, which further implies fully ripened state of climacteric fruit), and (2) climacteric peak of ethylene emitted from the climacteric peak is reached, and (1) and (2) occurs at the same time. The method 500 thereafter ends at 520.

**Example scenario:**

The disclosed system was utilized to determine natural ripening process of climacteric fruit, for example, banana which has a very high consumption rate across the globe, and hence estimate the natural ripening solution for a particular grade of banana named 'Cavendish Cultivar'.

Export grade bananas are generally picked hard and mature from the farm and artificially ripened to edible state using harmful chemical like industrial grade calcium carbide or ethophene within 2-3 days or placed in air-tight ethylene chambers for 10-12 days. During various stages of the natural ripening process, annotations were considered from banana experts from the fruit industry or published annotations based on pictures to classify different stages of fruit ripening.

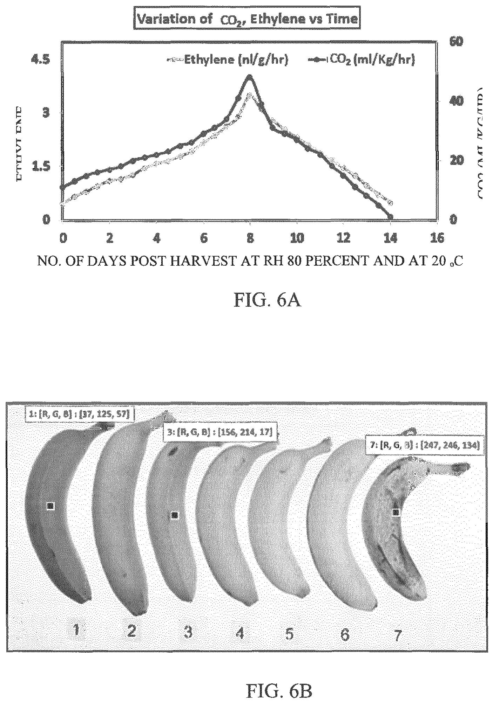

Ethylene is known as the fruit ripening hormone and different level of ethylene is produced by every climacteric fruit during different stages of its life cycle. As is illustrated in FIG. 6A, ethylene concentration gradually increases in any climacteric mature, unripe fruit, such as the case of a banana and triggers the fruit's metabolism, accelerating the ripening process naturally. The increase in ethylene concentration reaches a peak beyond which it starts to decrease. The ethylene concentration increases under controlled environment, increasing the ripening rate of the banana in pre-climacteric phase gradually de-greening the color of the banana to yellowish, followed by attaining a climacteric peak when the concentration of ethylene is maximum and the banana is completely ripened with aroma and colour changed to yellow with brown spots. Beyond the climacteric peak, ethylene concentration gradually decreases in the post climacteric phase when the fruit starts to over ripen and gets gradually degraded, turning to black in color.

Referring to FIG. 6B, life cycle of a natural ripening conditions for a hard green banana ripened fully in 8 days is presented below under temperature of 20° C and RH 80% is presented graphically. The natural ripening life cycle has been tested for different controlled environments and validated by means of expert annotations. The emitted ethylene follows a similar trend as of emitted CO2 from the banana over time under controlled environment. Emission of CO2 increases during the natural ripening of climacteric fruits till it approaches climacteric peak and then decreases when the fruit starts to over ripen similar to ethylene emission. The pictorial changes in the banana during its natural ripening phase is presented in FIG. 6B. As seen in FIG. 6A, the ripening process accelerates as increase in ethylene approaches the climacteric peak. The climacteric peak achieved was attained on the 8th day of the ripening process post-harvest which ensures the fruit is completely ripe and is ideal in terms of sweetness, taste, flavor and quality for human consumption and the same is validated as per expert annotations. As shown in FIG. 7A, when temperature is increased to 25∘C and a decreased RH of 75% the ripening process fastens and the banana reaches climacteric peak on the $4_{th}$day and the banana is completely ripe with RI as 8. However, ripening at lower humidity decreases the quality and flavor of the banana. Similarly, on decreasing the temperature and increasing the RH, slow down the ripening process and the same is depicted in FIG. 7B. When a banana at RI 1 is kept inside the enclosure under RH 85% and 18∘C, the banana attains RI 7 on the $10_{th}$day and becomes ideally ripe (FIG.7B). Further increase in humidity is capable of decelerating the ripening process further. When RH was increased to 95% maintaining the temperature at 18∘C, it takes 12 days for a banana to naturally ripen from RI 1 to RI 7 and attain the climacteric peak when it becomes ideal for consumption (FIG. 7C). The Cavendish Cultivar banana ripened at 18∘C and 95 % gives the maximum has the best quality in terms of sweetness, taste and flavor as compared to the other three naturally ripened bananas.

Another sample 'Cavendish Cultivar' banana was taken at RI 3. The RI was calculated based on RGB image analysis and validated with the RGB image annotation of the reported banana pictures [FIG. 7A]. As per FIG. 7A, the same Cavendish cultivar of banana at 20°C and 80% RH reaches RI 3 from RI 1 on the $3_{rd}$day and from RI 3 to RI 8 after 5 days (on the $8_{th}$day). Hence, under similar conditions, the banana taken at RI 3 should reach RI 7 after 5 days. Even the experimental results show the same (FIG. 7D). Hence we were able to correctly predict number of days needed by the sample banana to reach from RI 3 to RI 7 and validate the prediction based on experimental results.

Further, MM equations (1,2,3,4,5) were solved to develop a regression model for estimation of CO2 rate. The resultant regression equation w.r.t. $CO_2$ evolution with related correlation coefficients were derived using Gong and Corey Method to calculate gas composition over time. The parameters of the regression model based on $CO_2$emission are as follows $\alpha$= 82.314 ml kg-ih-i, $\varphi$= 11.346, yc = 71.124. The change of rate of gaseous concentration was calculated from first derivative of regression functions. The regression function corresponding to combination of uncompetitive and competitive equation (5) significantly matches with the experimental data ($R_2$> 0.98)andthe same is presented in FIG. 7E. Since the respiration rate model based on enzyme kinetics significantly matches with the experimental data, hence, the optimized model can be used to predict number of days that will be necessary to attain a particular RI from the current RI for natural ripening of a given climacteric fruit based on calculated respiration rate using the principles of Enzyme kinetics and MM equation for a particular environment condition.

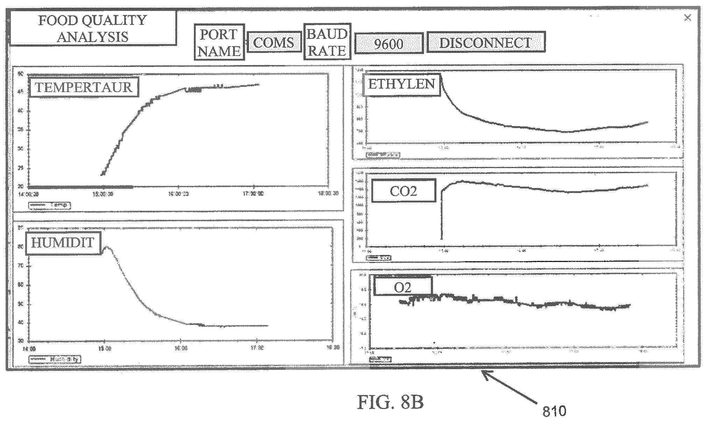

FIGS. 8A and 8B illustrates GUI 800, 810 respectively for online, real-time remote monitoring of the environment condition parameters in enclosure for management of ripening conditions of climacteric fruit, in accordance with an example embodiment. The GUI 800 is in form of a dashboard that shows real-time, continuous and dynamic data logging of the varying environment parameters over time. As the disclosed system is IoT enabled and allows online remote monitoring of all the sensory data along with high resolution images, thus enables the user to study the natural ripening process of the select fruit from hard, unripe stage to soft ripened state and estimate the optimal ripening solutions under controlled environment.

The written description describes the subject matter herein to enable any person skilled in the art to make and use the embodiments. The scope of the invention is defined by the claims.

Various embodiments disclose method and system for managing natural ripening of climacteric fruits. The disclosed system is capable of quantitative sensing measurement of the periodic variations of CO2, ethylene and consumed O2 within the custom enclosure under controlled environment conditions such as temperature, relative humidity and selective ventilation. The system enables estimating the optimal ripening conditions of a given climacteric fruit by recreating various environment conditions and varying them to achieve an estimate of the optimal conditions and quantitative data feeds. Various conditions can be recreated so as to dynamically able to take decisions for a supply chain application so that the fruit is made available from orchard to consumer in its ideal state.

The disclosed system can be used to evaluate optimal natural ripening solutions for any select climacteric fruit. The ripening conditions of the climacteric fruit can also be varied such as to follow the diurnal sinusoidal cyclic pattern in terms of light, temperature and relative humidity similar to as of morning, afternoon, evening and night to maintain natural environment to achieve effective natural ripening of climacteric fruits like banana. Moreover the ripening conditions such as Temperature, Relative Humidity and ventilation can be controlled and varied to achieve faster ripening or to slow down the ripening process for storage in store houses. Therefore, if the natural ripening conditions for any given climacteric fruit is estimated, the number of days required for the natural ripening can be determined and regulated as per requirement and usage can be planned accordingly.

The system finds its application in supply chain industry in terms of judicious and economically planned transportation and distribution of naturally ripened climacteric fruits to consumer end. Moreover, the system, being IoT enabled, allows online remote monitoring of naturally ripening climacteric fruits and thus ensures the ripening status for long distance transportation and thereby, opens up avenues for repurposing the climacteric fruit and hence reduce wastage. Supermarkets too can grade fruits according to their days to ripen in a quantitative manner & ease consumer dilemma while ensuring safe ripening.

The system is capable of estimating natural ripening of climacteric fruits and quantitatively sensing the emitted ethylene, CO2 and consumed O2 under controlled environment and selective ventilation, enables user to obtain natural ripening solutions for different types of climacteric fruit which can be ripened post-harvest off the plant prior to selling to consumers. Different climacteric fruits ripen at different rate depending upon the controlled variation of temperature, relative humidity, air circulation and emitted concentration of ethylene, CO2 and consumed O2. Hence, the system can be used to vary the temperature and relative humidity, ethylene gas levels and control the rate of air circulation inside the custom enclosure to study the effects of varying these parameters on the natural ripening process of different types of climacteric fruits with the aim to estimate the optimal ripening conditions of that particular climacteric fruit.

Moreover, considering the example of fruit processing and supply chain industry, the system is highly beneficial to plan transportation and distribution of ripe climacteric fruits to consumers. The system is capable of continuous synchronized sensing and recording of emitted ethylene, CO2 and consumed O2 in ppm levels inside the custom enclosure, under controlled temperature and relative humidity conditions from the sensory data along with automatic capturing of high resolution images. Combined quantitative monitoring of sensory data and captured high resolution images helps in studying the natural ripening process of various types of climacteric fruits and hence enables the user to estimate the optimal ripening condition in terms of required CO2, O2, ethylene and required number of days under user defined conditions such as light exposure, temperature and relative humidity along with cyclic variations of these conditions to mimic the actual diurnal pattern of nature. Technically, the optimized model can be used to predict number of days that will be necessary to attain a particular ripening index (RI) from the current RI for natural ripening of a given climacteric fruit based on calculated respiration rate using the principles of Enzyme kinetics and MM model for a particular environment condition.

Once the optimal ripening solution of a particular type of climacteric fruit plucked off the plant at a particular time, is estimated, the transportation and distribution of such fruits, post ripening, can be planned economically and judiciously and therefore reduce chances of damage due to transportation, over-ripening and wastage. For example, if the optimal ripening conditions and number of days necessary for the natural ripening inside the custom enclosure under controlled environment of an Alphonso mango plucked unripe at a certain maturity stage are known, then provisions can be made for controlled ripening of climacteric mangoes during the transportation and judiciously use the time of transportation for natural ripening, using the principles of the system. Also by varying the temperature and humidity inside the enclosure, required number of days for the natural ripening can be altered to match the transportation criteria and hence prevent over-ripening or under ripening of the fruits in the stipulated transportation time before selling it to the consumers. Moreover, the system being completely IoT enabled, allows remote access to the sensory data and high resolution images during transportation and hence allow timely updates on the ripening status of fruits and thus largely benefit the food processing and supply chain industry. Since real-time, remote monitoring is possible, decisions can be taken to avert losses due to over-ripening.

The system can also act as a product to be kept in supermarkets as natural ripening chambers for climacteric fruits that can influence customers on buying 100% safe and naturally ripened seasonal fruits without the use of any harmful chemicals. This can ensure increased consumption of healthy and naturally ripened climacteric fruits in daily diets which may retain the original nutritional value along with sweetness, odour, and flavour and nutrition quality.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A method for managing ripening conditions of climacteric fruit, comprising:

    obtaining levels of environment condition parameters associated with ripening of the climacteric fruit over time at periodic intervals by using an enclosure enclosing the climacteric fruit, via one or more hardware processors, the environment condition parameters comprising CO2 emitted, O2 consumed, Ethylene emitted, temperature and relative humidity measured within the enclosure, wherein the enclosure is integrated with mutually exclusive sensors for periodic and synchronized data logging corresponding to the climacteric fruit;

computing, via the one or more hardware processors, a respiration rate of the climacteric fruit based on the levels of the environment condition parameters and Michaelis Menten coefficients , wherein the Michaelis Menten coefficients comprise a Michaelis constant for O2 consumption (%), a Michaelis constant for competitive inhibition of O2 consumption by CO2 (%) and a Michaelis constant for the uncompetitive inhibition of O2 consumption by CO2 (%), wherein the respiration rate is expressed in terms of O2 consumption rate and CO2 production rate, the O2 consumption rate and CO2 production rate varying depending on environment and presence of inhibitors within the enclosure;

monitoring, via the one or more hardware processors, a level of Ethylene emitted in the enclosure to determine a climacteric peak of Ethylene for the climacteric fruit, the climacteric peak indicative of complete natural ripening of the climacteric fruit;

predicting, by a pre-trained artificial neural network (ANN) model, optimal ripening condition of the climacteric fruit in real time based on the respiration rate of the climacteric fruit and the climacteric peak of ethylene, via the one or more hardware processors, wherein the optimal ripening conditions comprises a number of days remaining to complete natural ripening of the climacteric fruit, wherein predicting the optimal ripening condition of the climacteric fruit comprises identifying stage of ripening of the climacteric fruit associated with a co-occurrence of the climacteric peak of ethylene emitted and a zero rate of change of the respiration rate; and

altering the environment condition parameters within the enclosure, based on the predicted optimal ripening condition, such that ripening kinetics of the climacteric fruit changes and the number of days remaining to complete natural ripening of the climacteric fruit is altered.

2. The method of claim 1, wherein altering the environment condition parameters comprises performing at least one of: varying the temperature and the relative humidity of the enclosure, ventilating excess CO2 and Ethylene when the levels of CO2 and ethylene reaches peak, wherein the enclosure is capable of providing selective ventilation.

3. The method of claim 1, further comprising training the ANN model for prediction based on a plurality of features, the plurality of features comprises CO2 emitted concentration over time, Ethylene emitted concentration over time, difference of O2 in the enclosure after a predefined interval, Temperature, Relative humidity, average Respiration rate, average temperature, average humidity, average ethylene rate, ethylene concentration at the climacteric peak, and CO2 concentration at the climacteric peak.

4. The method of claim 1, wherein predicting the optimal ripening condition of the climacteric fruit comprises identifying stage of ripening of the climacteric fruit associated with a co-occurrence of the climacteric peak of ethylene emitted and a zero rate of change of the respiration rate.

5. A system (300) for managing ripening conditions of climacteric fruit, comprising:

a memory (304) storing instructions;
one or more communication interfaces (306); and
one or more hardware processors (302) coupled to the memory (304) via the one or more communication interfaces (306), wherein the one or more hardware processors (302) are configured by the instructions to:

obtain levels of environment condition parameters associated with ripening of the climacteric fruit over time at periodic intervals by using an enclosure enclosing the climacteric fruit, the environment condition parameters comprising CO2 emitted, O2 consumed, Ethylene emitted, temperature and relative humidity measured within the enclosure, wherein the enclosure is integrated with mutually exclusive sensors for periodic and synchronized data logging corresponding to the climacteric fruit;
compute a respiration rate of the climacteric fruit based on the levels of the environment condition parameters and Michaelis Menten coefficients, wherein the Michaelis Menten coefficients comprise a Michaelis constant for O2 consumption (%), a Michaelis constant for competitive inhibition of O2 consumption by CO2 (%) and a Michaelis constant for the uncompetitive inhibition of O2 consumption by CO2 (%), wherein the respiration rate is expressed in terms of O2 consumption rate and CO2 production rate, the O2 consumption rate and CO2 production rate varying depending on environment and presence of inhibitors within the enclosure;
monitor a level of Ethylene emitted in the enclosure to determine a climacteric peak of Ethylene for the climacteric fruit, the climacteric peak indicative of complete natural ripening of the climacteric fruit;

predict, by a pre-trained artificial neural network (ANN) model, optimal ripening condition of the climacteric fruit in real-time based on the respiration rate of the climacteric fruit and the climacteric peak of ethylene, wherein the optimal ripening conditions comprises a number of days remaining to complete natural ripening of the climacteric fruit, wherein predicting the optimal ripening condition of the climacteric fruit comprises identifying stage of ripening of the climacteric fruit associated with a co-occurrence of the climacteric peak of ethylene emitted and a zero rate of change of the respiration rate; and

alter the environment condition parameters within the enclosure, based on the predicted optimal ripening condition, such that ripening kinetics of the climacteric fruit changes and the number of days remaining to complete natural ripening of the climacteric fruit is altered.

6. The system of claim 5, wherein the one or more hardware processors are further configured by the instructions to alter the environment condition parameters by performing at least one of: varying the temperature and the relative humidity of the enclosure, ventilating excess CO2 and Ethylene when the levels of CO2 and ethylene reaches peak, wherein the enclosure is capable of providing selective ventilation.

7. The system of claim 5, wherein the one or more hardware processors are further configured by the instructions to train the ANN model for prediction based on a plurality of features, the plurality of features comprises CO2 emitted concentration over time, Ethylene emitted concentration over time, difference of O2 in the enclosure after a predefined interval, Temperature, Relative humidity, average Respiration rate, average temperature, average humidity, average ethylene rate, ethylene concentration at the climacteric peak, and CO2 concentration at the climacteric peak.

**Patentansprüche**

1. Verfahren zum Verwalten von Reifungsbedingungen von klimakterischer Frucht, umfassend:

Erhalten von Niveaus von Umgebungsbedingungsparametern, die mit der Reifung der klimakterischen Frucht im Laufe der Zeit in periodischen Intervallen assoziiert sind, unter Verwendung eines Gehäuses, das die klimakterische Frucht umschließt, über einen oder mehrere Hardwareprozessoren, wobei die Umgebungsbedingungsparameter CO2 - Emission, O2 -Verbrauch, Ethylen-Emission, Temperatur und relative Feuchtigkeit, gemessen innerhalb des Gehäuses, umfassen, wobei das Gehäuse mit sich gegenseitig ausschließenden Sensoren zur periodischen und synchronisierten Datenerfassung, die der klimakterischen Frucht entsprechen, integriert ist;

Berechnen, über den einen oder die mehreren Hardwareprozessoren, einer Atmungsrate der klimakterischen Frucht basierend auf den Niveaus der Umgebungsbedingungsparameter und Michaelis-Menten-Koeffizienten, wobei die Michaelis-Menten-Koeffizienten eine Michaelis-Konstante für den O2 -Verbrauch (%), eine Michaelis-Konstante für die kompetitive Hemmung des O2 -Verbrauchs durch CO2 (%) und eine Michaelis-Konstante für die nicht-kompetitive Hemmung des O2 -Verbrauchs durch CO2 (%) umfassen, wobei die Atmungsrate in Form der O2 -Verbrauchsrate und der CO2 - Produktionsrate ausgedrückt wird, wobei die O2 -Verbrauchsrate und die CO2 -Produktionsrate in Abhängigkeit von der Umgebung und dem Vorhandensein von Inhibitoren innerhalb des Gehäuses variieren;

Überwachen, über den einen oder die mehreren Hardwareprozessoren, eines Niveaus von in dem Gehäuse emittiertem Ethylen, um einen klimakterischen Peak von Ethylen für die klimakterische Frucht zu bestimmen, wobei der klimakterische Peak eine vollständige natürliche Reifung der klimakterischen Frucht anzeigt;

Vorhersagen, durch ein vortrainiertes Modell eines künstlichen neuronalen Netzwerks (ANN), einer optimalen Reifungsbedingung der klimakterischen Frucht in Echtzeit basierend auf der Atmungsrate der klimakterischen Frucht und dem klimakterischen Peak von Ethylen, über den einen oder die mehreren Hardwareprozessoren, wobei die optimalen Reifungsbedingungen eine Anzahl von Tagen umfassen, die für eine vollständige natürliche Reifung der klimakterischen Frucht verbleiben, wobei das Vorhersagen der optimalen Reifungsbedingung der klimakterischen Frucht ein Identifizieren eines Reifungsstadiums der klimakterischen Frucht umfasst, das mit einem gleichzeitigen Auftreten des klimakterischen Peaks von emittiertem Ethylen und einer Nulländerungsrate der Atmungsrate assoziiert ist; und

Ändern der Umgebungsbedingungsparameter innerhalb des Gehäuses basierend auf der vorhergesagten optimalen Reifungsbedingung, sodass sich die Reifungskinetik der klimakterischen Frucht ändert und die Anzahl von Tagen, die für eine vollständige natürliche Reifung der klimakterischen Frucht verbleiben, geändert wird.

2. Verfahren nach Anspruch 1, wobei das Ändern der Umgebungsbedingungsparameter ein Durchführen von mindestens einem von Folgendem umfasst: Variieren der Temperatur und der relativen Feuchtigkeit des Gehäuses, Belüften von überschüssigem CO2 und Ethylen, wenn die Niveaus von CO2 und Ethylen einen Peak erreichen, wobei das Gehäuse in der Lage ist, eine selektive Belüftung bereitzustellen.

3. Verfahren nach Anspruch 1, ferner umfassend ein Trainieren des ANN-Modells zur Vorhersage basierend auf einer Vielzahl von Merkmalen, wobei die Vielzahl von Merkmalen eine CO2-emittierte Konzentration im Zeitverlauf, eine Ethylen-emittierte Konzentration im Zeitverlauf, eine Differenz von O2 in dem Gehäuse nach einem vordefinierten Intervall, Temperatur, relative Feuchtigkeit, durchschnittliche Atmungsrate, durchschnittliche Temperatur, durchschnittliche Feuchtigkeit, durchschnittliche Ethylenrate, Ethylenkonzentration an dem klimakterischen Peak und CO2 -Konzentration an dem klimakterischen Peak umfasst.

4. Verfahren nach Anspruch 1, wobei das Vorhersagen der optimalen Reifungsbedingung der klimakterischen Frucht ein Identifizieren eines Reifungsstadiums der klimakterischen Frucht umfasst, das mit einem gleichzeitigen Auftreten des klimakterischen Peaks von emittiertem Ethylen und einer Nulländerungsrate der Atmungsrate assoziiert ist.

5. System (300) zum Verwalten von Reifungsbedingungen von klimakterischer Frucht, umfassend:

einen Speicher (304), der Anweisungen speichert;
eine oder mehrere Kommunikationsschnittstellen (306); und
einen oder mehrere Hardwareprozessoren (302), die über die eine oder die mehreren Kommunikationsschnittstellen (306) an den Speicher (304) gekoppelt sind, wobei der eine oder die mehreren Hardwareprozessoren (302) durch die Anweisungen konfiguriert sind zum:

Erhalten von Niveaus von Umgebungsbedingungsparametern, die mit der Reifung der klimakterischen Frucht im Laufe der Zeit in periodischen Intervallen assoziiert sind, unter Verwendung eines Gehäuses, das die klimakterische Frucht umschließt, wobei die Umgebungsbedingungsparameter CO2 -Emission, O2 -Verbrauch, Ethylen-Emission, Temperatur und relative Feuchtigkeit, gemessen innerhalb des Gehäuses, umfassen, wobei das Gehäuse mit sich gegenseitig ausschließenden Sensoren zur periodischen und synchronisierten Datenerfassung, die der klimakterischen Frucht entsprechen, integriert ist;
Berechnen einer Atmungsrate der klimakterischen Frucht basierend auf den Niveaus der Umgebungsbedingungsparameter und Michaelis-Menten-Koeffizienten, wobei die Michaelis-Menten-Koeffizienten eine Michaelis-Konstante für den O2-Verbrauch (%), eine Michaelis-Konstante für die kompetitive Hemmung des O2 -Verbrauchs durch CO2 (%) und eine Michaelis-Konstante für die nicht-kompetitive Hemmung des O2 -Verbrauchs durch CO2 (%) umfassen, wobei die Atmungsrate in Form der O2 - Verbrauchsrate und der CO2 -Produktionsrate ausgedrückt wird, wobei die O2 -Verbrauchsrate und die CO2 -Produktionsrate in Abhängigkeit von der Umgebung und dem Vorhandensein von Inhibitoren innerhalb des Gehäuses variieren;
Überwachen eines Niveaus von in dem Gehäuse emittiertem Ethylen, um einen klimakterischen Peak von Ethylen für die klimakterische Frucht zu bestimmen, wobei der klimakterische Peak eine vollständige natürliche Reifung der klimakterischen Frucht anzeigt;
Vorhersagen, durch ein vortrainiertes Modell eines künstlichen neuronalen Netzwerks (ANN), einer optimalen Reifungsbedingung der klimakterischen Frucht in Echtzeit basierend auf der Atmungsrate der klimakterischen Frucht und dem klimakterischen Peak von Ethylen, wobei die optimalen Reifungsbedingungen eine Anzahl von Tagen umfassen, die für eine vollständige natürliche Reifung der klimakterischen Frucht verbleiben, wobei das Vorhersagen der optimalen Reifungsbedingung der klimakterischen Frucht ein Identifizieren eines Reifungsstadiums der klimakterischen Frucht umfasst, das mit einem gleichzeitigen Auftreten des klimakterischen Peaks von emittiertem Ethylen und einer Nulländerungsrate der Atmungsrate assoziiert ist; und
Ändern der Umgebungsbedingungsparameter innerhalb des Gehäuses basierend auf der vorhergesagten optimalen Reifungsbedingung, sodass sich die Reifungskinetik der klimakterischen Frucht ändert und die Anzahl von Tagen, die für eine vollständige natürliche Reifung der klimakterischen Frucht verbleiben, geändert wird.

6. System nach Anspruch 5, wobei der eine oder die mehreren Hardwareprozessoren ferner durch die Anweisungen konfiguriert sind, um die Umgebungsbedingungsparameter durch Durchführen von mindestens einem von Folgendem zu ändern: Variieren der Temperatur und der relativen Feuchtigkeit des Gehäuses, Belüften von überschüssigem CO2 und Ethylen, wenn die Niveaus von CO2 und Ethylen einen Peak erreichen, wobei das Gehäuse in der Lage ist, eine selektive Belüftung bereitzustellen.

**7.** System nach Anspruch 5, wobei der eine oder die mehreren Hardwareprozessoren ferner durch die Anweisungen konfiguriert sind, um das ANN-Modell zur Vorhersage basierend auf einer Vielzahl von Merkmalen zu trainieren, wobei die Vielzahl von Merkmalen eine CO2-emittierte Konzentration im Zeitverlauf, eine Ethylen-emittierte Konzentration im Zeitverlauf, eine Differenz von O2 in dem Gehäuse nach einem vordefinierten Intervall, Temperatur, relative Feuchtigkeit, durchschnittliche Atmungsrate, durchschnittliche Temperatur, durchschnittliche Feuchtigkeit, durchschnittliche Ethylenrate, Ethylenkonzentration an dem klimakterischen Peak und CO2 -Konzentration an dem klimakterischen Peak umfasst.

**Revendications**

**1.** Procédé de gestion de conditions de maturation de fruits climactériques, comprenant :

l'obtention de niveaux de paramètres de conditions environnementales associés à la maturation des fruits climactériques au fil du temps à intervalles périodiques en utilisant une enceinte renfermant les fruits climactériques, via un ou plusieurs processeurs matériel, les paramètres de conditions environnementales comprenant le CO2 émis, l'O2 consommé, l'éthylène émis, la température et l'humidité relative mesurées à l'intérieur de l'enceinte, dans lequel l'enceinte intègre des capteurs mutuellement exclusifs pour l'enregistrement chronologique de données périodiques et synchronisées correspondant aux fruits climactériques ;

le calcul, via les un ou plusieurs processeurs matériel, d'un taux de respiration des fruits climactériques sur la base des niveaux des paramètres de conditions environnementales et de coefficients de Michaelis Menten, dans lequel les coefficients de Michaelis Menten comprennent une constante de Michaelis pour la consommation d'O2 (%), une constante de Michaelis pour l'inhibition compétitive de consommation d'O2 par le CO2 (%) et une constante de Michaelis pour l'inhibition non-compétitive de consommation d'O2 par le CO2 (%), dans lequel le taux de respiration est exprimé en termes de taux de consommation d'O2 et de taux de production de CO2, le taux de consommation d'O2 et le taux de production de CO2 variant en fonction de l'environnement et de la présence d'inhibiteurs à l'intérieur de l'enceinte ;

la surveillance, via les un ou plusieurs processeurs matériel, d'un niveau d'éthylène émis dans l'enceinte pour déterminer un pic climactérique d'éthylène pour les fruit climactériques, le pic climactérique étant indicatif d'une maturation naturelle complète des fruits climactériques ;

la prédiction, par un modèle de réseau de neurones artificiels (RNA) préalablement formé, d'une condition de maturation optimale des fruits climactériques en temps réel sur la base du taux de respiration des fruit climactériques et du pic climactérique d'éthylène, via les un ou plusieurs processeurs matériel, dans lequel les conditions de maturation optimales comprennent un nombre de jours restants pour compléter la maturation naturelle des fruits climactériques, dans lequel la prédiction de la condition de maturation optimale des fruits climactériques comprend l'identification du stade de maturation des fruits climactériques associé à une cooccurrence du pic climactérique d'éthylène émis et d'un taux zéro de changement du taux de respiration ; et

la modification des paramètres de conditions environnementales à l'intérieur de l'enceinte, sur la base de la condition de maturation optimale prédite, de telle sorte que la cinétique de maturation des fruits climactériques change et que le nombre de jours restants pour compléter la maturation naturelle des fruits climactérique est modifié.

**2.** Procédé selon la revendication 1, dans lequel la modification des paramètres de conditions environnementales comprend la réalisation d'au moins une de : la variation de la température et de l'humidité relative de l'enceinte, la ventilation de l'excès de CO2 et d'éthylène lorsque les niveaux de CO2 et d'éthylène atteignent le pic, dans lequel l'enceinte est apte à fournir une ventilation sélective.

**3.** Procédé selon la revendication 1, comprenant en outre la formation du modèle RNA à la prédiction sur la base d'une pluralité de caractéristiques, la pluralité de caractéristiques comprend la concentration de CO2 émis au fil du temps, la concentration d'éthylène émis au fil du temps, la différence d'O2 dans l'enceinte après un intervalle prédéfini, la température, l'humidité relative, le taux de respiration moyen, la température moyenne, le taux d'éthylène moyen, la concentration d'éthylène au pic climactérique et la concentration de CO2 au pic climactérique.

**4.** Procédé selon la revendication 1, dans lequel la prédiction de la condition de maturation optimale des fruits climactériques comprend l'identification du stade de maturation des fruits climactériques associé à une cooccurrence du pic climactérique d'éthylène émis et d'un taux zéro du taux de respiration.

**5.** Système (300) de gestion de conditions de maturation de fruits climactériques, comprenant :

une mémoire (304) stockant des instructions ;

une ou plusieurs interfaces de communication (306) ; et

un ou plusieurs processeurs matériel (302) couplés à la mémoire (304) via les une ou plusieurs interfaces de communication (306), dans lequel les un ou plusieurs processeurs matériel (302) sont configurés par les instructions pour :

obtenir des niveaux de paramètres de conditions environnementales associés à la maturation des fruits climactériques au fil du temps à intervalles périodiques en utilisant une enceinte renfermant les fruits climactériques, les paramètres de conditions environnementales comprenant le CO2 émis, l'O2 consommé, l'éthylène émis, ka température et l'humidité relative mesurée à l'intérieur de l'enceinte, dans lequel l'enceinte intègre des capteurs mutuellement exclusifs pour l'enregistrement chronologique de données périodiques et synchronisées correspondant aux fruit climactériques ;

calculer un taux de respiration des fruits climactériques sur la base des niveaux des paramètres de conditions environnementales et de coefficients de Michaelis Menten, dans lequel les coefficients de Michaelis Menten comprennent une constante de Michaelis pour la consommation d'O2 (%), une constante de Michaelis pour l'inhibition compétitive de consommation d'O2 par le CO2 (%) et une constante de Michaelis pour l'inhibition non-compétitive de consommation d'O2 par le CO2 (%), dans lequel le taux de respiration est exprimé en termes de taux de consommation d'O2 et de taux de production de CO2, le taux de consommation d'O2 et le taux de production de CO2 variant en fonction de l'environnement et de la présence d'inhibiteurs à l'intérieur de l'enceinte ;

surveiller un niveau d'éthylène émis dans l'enceinte pour déterminer un pic climactérique d'éthylène pour les fruits climactériques, le pic climactérique étant indicatif d'une maturation naturelle complète des fruits climactériques ;

prédire, à l'aide d'un modèle de réseau de neurones artificiels (RNA) préalablement formé, une condition de maturation optimale des fruits climactériques en temps réel sur la base du taux de respiration des fruits climactériques et du pic climactérique d'éthylène, dans lequel les conditions de maturation optimales comprennent un nombre de jours restants pour compléter la maturation naturelle des fruits climactériques, dans lequel la prédiction de la condition de maturation optimale des fruits climactériques comprend l'identification du stade de maturation des fruits climactériques associé à une cooccurrence du pic climactérique d'éthylène émis et d'un taux zéro de changement du taux de respiration ; et

modifier les paramètres de conditions environnementales à l'intérieur de l'enceinte, sur la base de la condition de maturation optimale prédite, de telle sorte que la cinétique de maturation des fruits climactériques change et que le nombre de jours restants pour compléter la maturation naturelle des fruits climactériques est modifié.

6. Système selon la revendication 5, dans lequel les un ou plusieurs processeurs matériel sont en outre configurés par les instructions pour modifier les paramètres de conditions environnementales en réalisant au moins une parmi : une variation de la température et de l'humidité relative de l'enceinte, une ventilation de l'excès de CO2 et d'éthylène lorsque les niveaux de CO2 et d'éthylène atteignent le pic, dans lequel l'enceinte est apte à fournir une ventilation sélective.

7. Système selon la revendication 5, dans lequel les un ou plusieurs processeurs matériel sont en outre configurés par les instructions pour former le modèle RNA à la prédiction sur la base d'une pluralité de caractéristiques, la pluralité de caractéristiques comprend la concentration de CO2 émis au fil du temps, la concentration d'éthylène émis au fil du temps, la différence d'O2 dans l'enceinte après un intervalle prédéfini, la température, l'humidité relative, le taux de respiration moyen, la température moyenne, l'humidité moyenne, le taux d'éthylène moyen, la concentration d'éthylène au pic climactérique et la concentration de CO2 au pic climactérique.

FIG. 1

FIG. 2

```
┌─────────────────────────────────────────────────────────────────────┐
│  ┌──────────────────────────────────┐        308        300          │
│  │        MEMORY 304                │    ↑                            │
│  │  ┌────────────────────────────┐  │    ║    ┌──────────────────┐   │
│  │  │      MODULES 320           │  │    ║    │      306         │   │
│  │  └────────────────────────────┘  │ ⇕  ║    │ USER INTERFACE   │   │
│  │  ┌────────────────────────────┐  │    ║    └──────────────────┘   │
│  │  │   REPOSITORY 340           │  │    ║                           │
│  │  │  ┌──────┐   ┌──────┐       │  │ ⇕  ║                           │
│  │  │  │ 342  │   │ 344  │       │  │    ║    ┌──────────────────┐   │
│  │  │  └──────┘   └──────┘       │  │    ║    │      302         │   │
│  │  │  ┌──────┐   ┌──────┐       │  │ ⇕  ║    │   PROCESSOR      │   │
│  │  │  │ 346  │   │ 348  │       │  │    ↓    └──────────────────┘   │
│  │  │  └──────┘   └──────┘       │  │                               │
│  │  └────────────────────────────┘  │                               │
│  └──────────────────────────────────┘                               │
└─────────────────────────────────────────────────────────────────────┘
```

FIG. 3

| OBTAIN LEVELS OF ENVIRONMENT CONDITION PARAMETERS ASSOCIATED WITH RIPENING OF THE CLIMACTERIC FRUIT OVER TIME AT PERIODIC INTERVALS BY USING AN ENCLOSURE ENCLOSING THE CLIMACTERIC FRUIT | 402 |

↓

| COMPUTE A RESPIRATION RATE OF THE CLIMACTERIC FRUIT BASED AT LEAST ON THE LEVELS OF THE ENVIRONMENT CONDITION PARAMETERS USING MICHAELIS MENTEN KINETICS MODEL | 404 |

↓

| MONITOR A LEVEL OF ETHYLENE EMITTED IN THE ENCLOSURE TO DETERMINE A CLIMACTERIC PEAK OF ETHYLENE FOR THE CLIMACTERIC FRUIT, THE CLIMACTERIC PEAK INDICATIVE OF COMPLETE NATURAL RIPENING OF THE CLIMACTERIC FRUIT | 406 |

↓

| PREDICT, BY A TRAINED ARTIFICIAL NEURAL NETWORK (ANN) MODEL, OPTIMAL RIPENING CONDITION OF THE CLIMACTERIC FRUIT BASED ON THE RESPIRATION RATE OF THE CLIMACTERIC FRUIT AND THE CLIMACTERIC PEAK OF ETHYLENE, WHEREIN THE OPTIMAL RIPENING CONDITIONS COMPRISES AT LEAST A NUMBER OF DAYS REMAINING TO COMPLETE NATURAL RIPENING OF THE CLIMACTERIC FRUIT | 408 |

FIG. 4

400

START — 502

MEASURE ENVIRONMENT CONDITION PARAMETERS SYNCHRONIZED CO2, DIFFERENCE OF O2, ETHYLENE, TEMPERATURE, RELATIVE HUMIDITY OVER TIME AT PERIODIC INTERVALS — 504

CALCULATE THE RESPIRATION RATE OF THE CLIMACTERIC FRUIT USING PREVIOUSLY COMPUTED COEFFICIENTS IN THE MICHAELIS MENTEN KINETICS MODEL FOR THE CLIMACTERIC FRUIT AND THE ENVIRONMNTAL CONDITION PARAMETERS — 506

COMPUTE RATE OF CHANGE OF RESPIRATION RATE — 508

MONITOR LEVEL OF ETHYLENE IN THE ENCLOSURE TO DETECT IF THE LEVEL OF ETHYLENE IS PEAKED — 514

IS RATE OF CHANGE OF RESPIRATION RATE EQUAL TO ZERO — 510

NO

YES

CLIMACTERIC PEAK OF ETHYLENE REACHED? — 516

NO

YES

YES

CO2 EMISSION IS HIGHEST — 512

CLIMACTERIC PEAK OF ETHYLENE IS ACHIEVED AND AN OPTIMAL RIPENING CONDITIONS FOR THE CORRESPONDING CLIMACTERIC FRUIT IS REACHED — 518

STOP — 520

FIG. 5

500

FIG. 6A

FIG. 6B

FIG. 7A

FIG. 7B

FIG. 7C

FIG. 7D

FIG. 7E

FIG. 8A

800

FIG. 8B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 201921008382

- IN 201821040783

**Non-patent literature cited in the description**

- **NAYARA CANTERO BARBOSA et al.** *Modeling the respiration rate of Golden papayas stored under different atmosphere conditions at room temperature*

- **SUNNY GEORGE GWANPUA et al.** *Modelling ethylene regulated changes in 'Hass' avocado quality*